# EUROPEAN PATENT SPECIFICATION

(11) **EP 4 135 614 B1**
(45) Date of publication and mention of the grant of the patent: **29.04.2026**
(21) Application number: 21717464.8
(22) Date of filing: 13.04.2021
(51) Int. Cl.: A61B 34/10, A61B 17/16, A61B 34/20, A61B 17/17

(54) **SYSTEM FOR DETERMINING A SAFETY CRITERION DURING AN AUTONOMOUS MANIPULATION OF A SURGICAL TOOL BY A ROBOTIC SYSTEM TO TREAT AN ANATOMICAL STRUCTURE**
SYSTEM ZUR BESTIMMUNG EINES SICHERHEITSKRITERIUMS WÄHREND EINER AUTONOMEN MANIPULATION EINES CHIRURGISCHEN WERKZEUGS DURCH EIN ROBOTERSYSTEM ZUR BEHANDLUNG EINER ANATOMISCHEN STRUKTUR
SYSTÈME DE DÉTERMINATION D'UN CRITÈRE DE SÉCURITÉ LORS D'UNE MANIPULATION AUTONOME D'UN INSTRUMENT CHIRURGICAL PAR UN SYSTÈME ROBOTIQUE POUR TRAITER UNE STRUCTURE ANATOMIQUE

(30) Priority: 16.04.2020 EP 20315184
(43) Date of publication of application: 22.02.2023
(73) Proprietor: Ecential Robotics, 38610 Gieres (FR)
(72) Inventor: LAVALLÉE, Stéphane, 38410 SAINT-MARTIN-D'URIAGE (FR); ARMAND, David, 38120 SAINT EGREVE (FR); CHABANAS, Laurence, 38830 CRETS EN BELLEDONNE (FR)
(74) Representative: Regimbeau
(86) International application number: PCT/EP2021/059589
(87) International publication number: WO 2021/209466

(56) References cited:
- US-A1- 2017 020 630
- US-A1- 2018 021 597

## Description

### TECHNICAL FIELD

The present disclosure relates to a method and a system for determining a safety criterion during an autonomous manipulation of a surgical tool by a robotic system to treat an anatomical structure.

### BACKGROUND OF THE INVENTION

Surgical robotic arms are already used for assisting a user (e.g. a surgeon) during a surgical intervention.

For example, in spine surgery, the user may have to implant one or several screws into at least one vertebra. The robotic arm may assist the user by holding a drill guide, aligning and maintaining the drill guide according to a linear trajectory planned on a 3D image that has been registered with the patient position. The user may thus use a handheld drill passing through the drill guide held by the robotic arm to drill a hole intended to receive the screw in a vertebra along the planned trajectory, the user can subsequently insert a screwdriver in the drill guide and insert a screw according to the desired trajectory.

Furthermore, more advanced robotic systems (called "active robotic systems") may be configured to autonomously manipulate a surgical tool according to a planned trajectory to treat an anatomical structure. For example, a drilling trajectory into a pedicle may be planned by the surgeon on a 3D image acquired prior to the surgical procedure, and the robotic system may autonomously carry out said drilling.

To that end, both the surgical tool and the anatomical structure may comprise a tracker rigidly attached thereto, each tracker being tracked by a localization system. Both the anatomical structure and the surgical tool can be localized, which allows determining in real time the relative position of the surgical tool relative to the anatomical structure to be treated. In this regard, the use of a localization system that can localize the trackers in real time (i.e. with high frequency, low latency) may be used to carry out such autonomous manipulation.

In the state of the art, Cascination (Bern, Switzerland) has developed an active robotic system used to drill a bone to place cochlear implants [1].

In some circumstances, a tracker may be rigidly attached to a part of the robotic system and not to the surgical tool directly, allowing indirect localization of the tool based on knowledge at any time of the kinematic model of the robotic system between the tracker and the tool.

However, the autonomous manipulation by a robotic system of a surgical tool for treating an anatomical structure comes with important safety issues.

Indeed, after the planning of the trajectory of the surgical tool, the surgeon does not act in a direct manner to execute the planned surgical procedure.

Moreover, the tracker attached to the anatomical structure may move, e.g. as a result of an involuntary shock or push applied to it. Or the tracker attached to the surgical tool may be off its calibration. Or any software error may lead to a geometric error leading to a false position of the autonomous robotic system while it executes its planned trajectory. In a similar fashion, a tracker may be defective for various reasons (e.g. blood spilled on it in case of optical tracking, electromagnetic disturbances in case of electromagnetic tracking), resulting in erroneous tracking.

In such cases, since the planned trajectory of the surgical tool is defined relative to a coordinate system of the anatomical structure attached to the tracker of the anatomical structure, the surgical tool will not be manipulated according to the originally targeted trajectory but according to an erroneous one.

Some surgical robotic systems in the state of the art use redundant sensors to avoid large mistakes in the behavior of the robotic system, such as redundant encoders on each robot axis, force sensors on the robot axis or robot shell, but this may not be sufficient to solve the issues mentioned above, especially when considering small deviations of a few millimeters with respect to the planned trajectory that are not easy to detect but that could still create severe damages to the patient.

It is also known to acquire X-ray images using conventional fluoroscopy imaging devices and check visually that the position of the surgical tool seems to be in the right position, but such a visual method is prone to errors and false interpretation by the surgeon; besides, it is not automatic and does not provide a quantitative measurement that can be used by the robotic system to stop the robotic arm for example, or to react quickly.

Document US 2017/020630 relates to a surgical system comprising an X-ray imaging system and a robotic system and discloses a method for registration of digital medical images. The method includes the step of storing a 3D digital medical image having a 3D anatomical feature and a first coordinate system and storing a 2D digital medical image having a 2D anatomical feature and a second coordinate system. The method further includes the steps of storing a placement of a digital medical object on the 3D digital medical image and the 2D digital medical image and generating a simulated 2D digital medical image from the 3D digital medical image, wherein the simulated 2D digital medical image comprises a simulated 2D anatomical feature corresponding to the 3D anatomical feature. The 2D anatomical feature is compared with the simulated 2D anatomical feature until a match is reached and a registration of the first coordinate system with the second coordinate system based on the match is determined.

Document US 2018/021597 relates to an X-ray imaging system and discloses a data processing method performed by a computer for monitoring the position of a patient, comprising the steps of: acquiring a 3D image dataset of the patient; acquiring an initial real image of the patient from a medical imaging system having a known and fixed location in space, thus defining a known viewing direction of the initial real image in space, the real image being taken at a first point in time; -performing 2D/3D registration by calculating a simulated image from the 3D image dataset which matches the initial real image best, the simulated image having a viewing direction onto the 3D image dataset; storing an initial similarity measure value for the pair of the initial real image and the calculated simulated image; -acquiring a subsequent real image from the medical imaging system taken at a second point in time later than the first point in time; calculating a subsequent similarity measure value for the pair of the subsequent real image and the calculated simulated image; and outputting an indication signal if the difference between the initial similarity measure value and the subsequent similarity measure value fulfills a predetermined criterion.

### SUMMARY OF THE INVENTION

For safety reasons, it is thus desirable to determine automatically a safety criterion that indicates whether the surgical tool is exactly on the desired and planned trajectory or not.

A goal of the invention is to define a control unit configured for determining a safety criterion during an autonomous manipulation by a robotic system of a surgical tool to treat an anatomical structure.

The invention relates a control unit as claimed in claim 1. Additional features of the control unit are defined in dependent claims.

The disclosure relates to a method (not claimed) for determining a safety criterion during an autonomous manipulation of a surgical tool by a robotic system to treat an anatomical structure according to a planned trajectory in a 3D image, said 3D image being registered with a patient tracker, and the robotic system being servo-controlled on the movements of the patient tracker, the method comprising:
a. acquiring at least one 2D X-ray image containing the anatomical structure and the surgical tool by an X-ray imaging system, and for each at least one 2D X-ray acquisition:
   i. synchronously localizing the surgical tool and the patient tracker to determine the position of the surgical tool relative to said 3D image,
   ii. registering the 2D X-ray image with the 3D image in a region of interest around the anatomical structure,
   iii. generating a projection onto the 2D X-ray image of a model of the surgical tool in its position relative to the 3D image computed in step (i) ('projected localized position'),
   iv. determining a real position of the surgical tool on the 2D X-ray image ('real position'),
b. determining a safety criterion from a similarity information between each real position and each projected localized position of the surgical tool on the at least one 2D X-ray image.

In some embodiments, steps (a) and (b) are repeated several times all along the autonomous manipulation of the surgical tool by the robotic system.

In some embodiments, steps (a) and (b) are first applied to at least two 2D X-ray images, and then applied to only one 2D X-ray image.

In some embodiments, the localization of the surgical tool in step (i) is determined by a tracker rigidly fixed to said surgical tool.

In some embodiments, the localization of the surgical tool in step (i) is determined by a tracker rigidly fixed to a part of the robotic system and the known kinematic model of the robotic system between said tracker and the surgical tool at the time of localization.

In some embodiments, step (a) comprises computing at least one optimal orientation of the X-ray imaging system relative to either the surgical tool or the anatomical structure and acquiring the at least one 2D X-ray image with said at least one optimal orientation of the X-ray imaging system.

In some embodiments, each one of steps (a) through (b) involves at least two 2D X-ray images and step (b) comprises computing a global safety criterion as a function of each similarity information computed for each 2D X-ray image.

Said function may be the maximum function.

In some embodiments, in step (i), the robotic system is in a fixed position with respect to the anatomical structure, on the planned trajectory, between the acquisitions of the at least two 2D X-ray images.

In other embodiments, in step (i), the robotic system is mobile with respect to the anatomical structure along its planned trajectory between the acquisitions of the at least two 2D X-ray images.

In some embodiments, the registration process in step (ii) is initiated by a known estimation of the position of the X-ray imaging system.

Said position of the X-ray imaging system may be determined by a tracker rigidly attached to said X-ray imaging system.

Alternatively, the X-ray imaging system may be motorized, and said position of said motorized X-ray imaging system is determined by its motor encoders values.

In some embodiments, the similarity information is the distance between the longitudinal axis corresponding to the real position of the surgical tool and the longitudinal axis corresponding to its projected localized position on a plane perpendicular to a predetermined point of interest along the planned trajectory of the surgical tool.

In some embodiments, the at least one 2D X-ray acquisition is performed with a collimation of the x-ray beam on the region of interest around the anatomical structure.

In some embodiments, the planned trajectory is a 3D complex path of a burr to resect a part of a bone.

The disclosure also relates to a method (not claimed) for controlling a robotic system, comprising the following steps:
- applying step (a) and (b) of the above described method in a given state of the robotic system,
- acquiring one new 2D X-ray image containing the anatomical structure and the surgical tool by an X-ray imaging system, said X-ray imaging system being in the same position as in the last 2D X-ray acquisition,
- comparing the new 2D X-ray image with the last acquired 2D X-ray image using a 2D/2D registration method providing a similarity criterion between the two registered 2D X-ray image,
- if the similarity criterion is below a predetermined threshold, continue manipulating the tool with the robotic system until the robotic system has reached its final position.
- if the similarity criterion is above said predetermined threshold, apply again the method.

The invention also relates to a system for an autonomous manipulation of a surgical tool to treat an anatomical structure according to a planned trajectory in a 3D image, said 3D image being registered with a patient tracker, said system comprising:
- a robotic system servo-controlled on the movements of the patient tracker, configured to manipulate the surgical tool according to the planned trajectory,
- an X-ray imaging system configured to acquire at least one 2D X-ray image containing the anatomical structure and the surgical tool,
- a localization system adapted to measure relative positions of the patient tracker and of a tracker rigidly fixed to the surgical tool or to a part of the robotic system,
- the above-described control unit, which is coupled to the robotic system, the X-ray imaging system and the localization system, configured to implement the following method:
   a. for each at least one 2D X-ray image (I_{2D}) acquired by the X-ray imaging system (2):
      i. obtaining synchronous localization measurement of the surgical tool and the patient tracker by the localization system to determine the position of the surgical tool relative to said 3D image,
      ii. registering the 2D X-ray image with the 3D image in a region of interest around the anatomical structure,
      iii. generating a projection onto the 2D X-ray image of a model of the surgical tool in its position relative to the 3D image computed in step (i) ('projected localized position'),
      iv. determining a real position of the surgical tool on the 2D X-ray image ('real position'),
   (b) determining a safety criterion from a similarity information between each real position and each projected localized position of the surgical tool on the at least one 2D X-ray image.

In some embodiments, the control unit is configured to repeat steps (a) and (b) several times all along the autonomous manipulation of the surgical tool by the robotic system.

In particular, the control unit may be configured to first apply steps (a) and (b) to at least two 2D X-ray images, and then apply steps (a) and (b) to only one 2D X-ray image.

In some embodiments, the system further comprises a tracker rigidly fixed to the surgical tool and adapted to be tracked by the localization system to provide to the control unit localization measurement of the surgical tool.

In some embodiments, the system further comprises a tracker rigidly fixed to a part of the robotic system and adapted to be tracked by the localization system, the control unit being configured to determine a localization of the surgical tool from localization measurement of the tracker rigidly fixed to the part of the robotic system and from a known kinematic model of the robotic system between said tracker and the surgical tool at the time of localization.

In some embodiments, the control unit is configured to compute in step (a) at least one optimal orientation of the X-ray imaging system relative to either the surgical tool or the anatomical structure and to control the X-ray imaging system to acquire the at least one 2D X-ray image with said at least one optimal orientation.

In some embodiments, the control unit is configured to apply each one of steps (a) through (b) to at least two 2D X-ray images and to compute in step (b) a global safety criterion as a function of each similarity information computed for each 2D X-ray image.

Said function may be the maximum function.

In some embodiments, the control unit is configured to initiate the registration process in step (ii) by a known estimation of the position of the X-ray imaging system.

The control unit may be configured to determine said position of the X-ray imaging system by tracking by the localization system a tracker rigidly attached to said X-ray imaging system.

Alternatively, the X-ray imaging system may be motorized, and the control unit may be configured to determine said position of said motorized X-ray imaging system by its motor encoders values.

In some embodiments, the control unit is configured to determine the similarity information as the distance between the longitudinal axis corresponding to the real position of the surgical tool and the longitudinal axis corresponding to its projected localized position on a plane perpendicular to a predetermined point of interest along the planned trajectory of the surgical tool.

In some embodiments, the X-ray imaging system is configured to perform at least one 2D X-ray acquisition with a collimation of the x-ray beam on the region of interest around the anatomical structure.

### BRIEF DESCRIPTION OF THE DRAWINGS

Further features and advantages of the invention will appear in the following detailed description, based on the appended drawings wherein:
- FIG. 1 illustrates a surgical scene including the system according to the invention;
- FIG. 2 illustrates the robotic system and the trackers used to implement the method;
- FIG. 3 illustrates the acquisition of a plurality of 2D X-ray images including the anatomical structure and the surgical tool;
- FIG. 4 illustrates a method for registering the 3D image with the 2D X-ray image by iteratively projecting the anatomical structure in the 3D image onto a plane of the X-ray detector and minimizing the error in the shape of the anatomical structure between the projection and the 2D X-ray image;
- FIG. 5 illustrates the projection of the localized position of the surgical tool onto the 2D X-ray image and the determination of the real position of the surgical tool on the 2D X-ray image;
- FIG. 6 schematically illustrates a similarity information;
- FIG. 7 schematically illustrates the method in an application for drilling a linear trajectory in a spine pedicle
- FIG. 8 schematically illustrates the method in an application for burring a bone;
- FIG. 9 is a schematic example of the management of the lags to minimize the time necessary to detect an error.

Reference signs identical from one figure to another one designate similar components.

### DETAILED DESCRIPTION OF EMBODIMENTS

The invention defined in the appended claims uses an X-ray imaging system producing 2D X-ray images, a 3D image that can be produced by said X-ray imaging system or by another imaging system, a robotic system that holds a surgical tool, and a localization system, such as presented below.

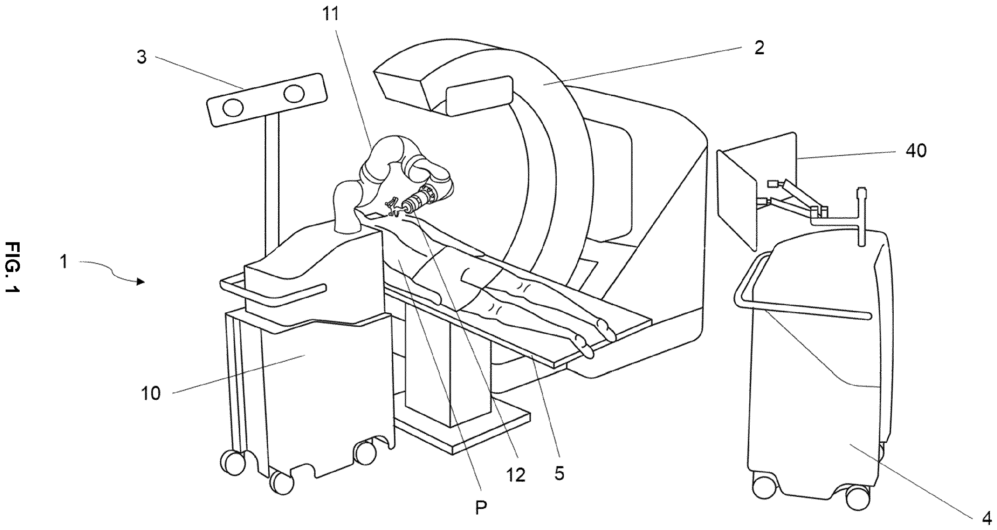

FIG. 1 illustrates a surgical scene comprising said systems in order to carry out a surgical intervention to treat an anatomical structure.

A patient P lies on an operating table 5.

The surgical system 1 comprises a base 10 and a robotic arm 11 carrying a powered surgical tool and is placed in the vicinity of the operating table.

The X-ray imaging system 2 is also placed in the vicinity of the operating table, to acquire 2D X-ray images of the anatomical structure to be treated.

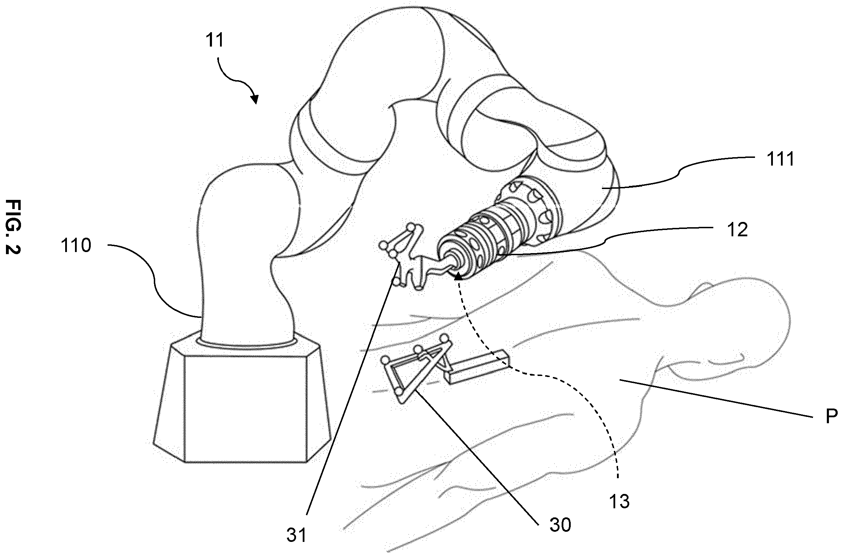

As shown in FIG. 2, the patient and the surgical system comprise at least one respective tracker rigidly attached thereto, said trackers being localized by a localization system 3.

A control unit 4 is coupled to the robotic system (in particular, to a controller of the robotic system) and the X-ray imaging system. The control unit comprises at least one processor configured to implement algorithms designed to carry out the method that will be described below. In particular, the control unit may be configured to perform the following steps: receive the 3D image (whether acquired by the X-ray imaging system 2 or by another system), receive 2D X-ray images acquired by the X-ray imaging system, receive a planning done on the 3D image or allow a user planning the surgical intervention on the 3D image, compute a trajectory of the robotic arm to follow the planning, compute relative position of the trackers based on data received from the localization system, control the robotic system to execute the computed trajectory taking into account the computed relative positions of the trackers. In some embodiments, the control unit may also be configured to trigger 2D X-ray image acquisitions.

The control unit may also be coupled to a user interface 40. The user interface may include one or more screens.

### X-ray imaging system

The X-ray imaging system comprises at least one X-ray source and at least one X-ray image detector. The X-ray imaging system produces at least one 2D X-ray image that is the result of a conical projection of a patient anatomy, wherein the tip of the cone is approximately the central point of the X-ray source and the basis of the cone is approximately the portion of the X-ray image detector that is reached by X-ray beams that have been collimated in a given shape and orientation.

For example, the X-ray imaging system can be a conventional C-arm, or any Cone-Beam Computed Tomography (CBCT), such as the Surgivisio device (Surgivisio, Gieres, France), or Vision FD Vario 3D (Ziehm), CIOS Spin Mobile 3D (Siemens), Airo (Stryker), Loop-X (Brainlab), O-arm (Medtronic).

A conventional C-arm is designed to allow the X-ray source and X-ray detector to rotate along a C-shaped gantry while obtaining projection images of the patient placed between the X-ray source and the X-ray detector of the gantry.

A CBCT has a mobile X-ray source and a mobile X-ray image detector, wherein the X-ray source and the X-ray image detector have motorized motions, moving together or independently. A CBCT can have a C-arm shape or an O-arm shape. It can be used to acquire a set of 2D X-ray images over approximately 180° of orbital rotation that can be combined with translations and from which a 3D image can be reconstructed using tomography algorithms or tomosynthesis algorithms.

The X-ray imaging system may be motorized, notably the C-shaped arm may comprise motors allowing movement horizontally, vertically and around the swivel axes, so that 2D X-ray images of the patient are produced from almost any angle. Each motor is associated to an encoder that provides at any time the relative position of the medical imaging system with respect to a reference position. When a 2D X-ray image is acquired, the corresponding position of the imaging system is recorded. Thus, each 2D image is recorded in the referential of the imaging system.

In some embodiments, a 3D image of the patient may be obtained during the surgery, using the X-ray imaging system itself if it is a CBCT. Said 3D image is registered with respect to a tracker attached to the anatomical structure using known methods of calibration and navigation.

In other embodiments, the 3D image may be acquired prior to surgery using a Computed Tomography (CT) device or another CBCT device. Said 3D image of the patient is registered with respect to the tracker attached to the anatomical structure using a 3D registration method that can use many techniques, such as (i) a collection of surface points using the localizing system and their fitting on the anatomical structure like in the technique provided by 7D Surgical (North York, Canada), or (ii) the acquisition of 2D X-ray images calibrated with respect to the tracker attached to the anatomical structure and used for registration with the 3D image like in the technique provided in the Mazor X robotic system (Medtronic), or (iii) any registration technique using localized ultrasound images, fiducials, anatomical points and the like.

### Robotic system

A robotic system 1, in the sense of the present disclosure, may comprise, with reference to FIGS. 1 and 2:
- a base 10, which may be a movable cart (as shown in FIG. 1), or may be attached to the operating table (this embodiment has not been illustrated),
- a robotic arm 11 having a proximal end 110 extending from the base, and a distal end 111 opposite to the proximal end,
- an end-effector 12 attached at the distal end 111 of the robotic arm, that may comprise a hand grip and a tool holder,
- a controller configured to controllably move the robotic arm according to a planned trajectory.

The robotic arm comprises a plurality of degrees of freedom in translation and/or rotation. Usually, the robotic arm comprises at least five, and preferably six or seven motorized degrees of freedom. To that end, the robotic arm comprises a plurality of articulated segments driven by motors. A robotic arm can be for example the LBR Med^{™} robot provided by KUKA (Germany). Said robotic arm can be controlled in an autonomous mode according to desired targets and trajectories, or it can be manipulated using a collaborative mode (cobot), or it can be telemanipulated using a master control device, and a combination of these different modes can be used on the same robotic system.

The robotic system is active in the sense that it holds and moves a powered surgical tool that interacts directly with the anatomical structure, contrary to a passive robotic system that holds a guide in a predefined position with respect to the anatomical structure into which a powered surgical tool is inserted by a surgeon. For example, a powered drill is mounted on the robotic arm tool holder and actively drills the bone along a predefined path until an end-point of a selected linear trajectory is reached.

The surgical tool is not illustrated in FIG. 2 but its position is represented schematically by reference 13.

Said powered surgical tool can be a powered drill, a powered saw, a powered burr or mill, an ultrasonic milling device, a radiofrequency or microwave or cryogenics ablation needle, or any device that can interact with the anatomical structure to be treated. For example, a powered burr can be used to remove a volume of bone where a tumor has been detected, the robotic system being controlled to have the burr tip execute a 3D complex path trajectory corresponding to the bone volume to be removed.

### Localization system

A localization system is used that can localize the three parameters of position and the three parameters of orientation of any tracker mounted rigidly on anatomical structures such as bones, or devices, such as surgical tools or a subsystem of the robotic system. Said localization system 3 can be an optical system (such as Polaris device from NDI, Canada), as in the embodiment illustrated in FIG. 1, or an electromagnetic system (such as Aurora from NDI, Canada), or any combination of optical, electromagnetic, ultrasonic, inertia measurement devices and sensors, or passive electro-mechanical arms with encoders.

A first tracker 30 may be rigidly attached to a base structure that is assumed to be fixed with respect to the anatomical structure to be treated, therefore said first tracker is supposed to be fixed with respect to the anatomical structure. Said base structure can be the anatomical structure itself, or an adjacent structure, or any mechanical fixation fixed to the patient or to the operating table if the motions between said base structure and the anatomical structure to be treated can be neglected for the required accuracy.

A second tracker 31 is rigidly attached to the surgical tool manipulated by the robotic arm or to any part of the robotic system itself, such as the base. In the latter case, the kinematic model of the robotic arm is known for any position of the robotic arm using its encoders values and therefore by a simple combination, the position and orientation of the surgical tool is known in a coordinate system attached to the second tracker at any time.

### Anatomical structure

The anatomical structure to be treated is usually a bone that may be drilled, burred, and/or milled in order to place an implant or to free some space for any clinical reason.

The method may be applied successively to several parts of one bone or to several bones. For example, the method may be used for placing screws in both pedicles of several vertebrae.

### Method

The robotic system may be operated as follows.

At the beginning of surgery, the patient is equipped with a first tracker 30 (called "patient tracker") detectable by the localization system 3.

A 3D image is acquired either at the beginning of surgery using the X-ray imaging system itself (CBCT) or prior to surgery with another imaging system (CT or CBCT), and as described above said 3D image is registered with respect to the patient tracker.

A plurality of trajectories of the surgical tool are planned in the 3D image. For that purpose, a surgical planning software is used to define surgical targets interactively or automatically in the 3D image. In the rest of the description, only one trajectory is considered, and the method is repeated for each trajectory.

The robotic system, which may be mobile on the wheels of the cart forming the base of the robotic arm, is brought near the surgical table.

The robotic system is equipped with a powered surgical tool.

As shown on FIG. 2, a second tracker 31 (called "robot tracker") is mounted on the robotic arm, on the surgical tool directly or on any sub-system of the robotic system. The calibration of the robot tracker with respect to the surgical tool can use several known calibration methods.

In a preferred embodiment, the position of the robot tracker on the surgical tool is reproducible and always the same, and a localized pointer is used to check that a particular point of the surgical tool has precisely the expected coordinates with respect to the robot tracker.

In another preferred embodiment, the localized pointer is used to digitize at least three precisely defined points on the surgical tool and a point-based calibration is applied.

The robotic system is then moved manually (cobot) or automatically, to align the surgical tool to the planned trajectory, until it reaches an entry point on the bone surface. The robotic system is then servo-controlled on the movements of the patient tracker in order to maintain alignment with the entry point position on the bone, compensating for any motions of the bone due to patient's breathing or any mechanical interactions.

At least one 2D X-ray image containing the anatomical structure and the surgical tool is acquired using the X-ray imaging system.

In a preferred embodiment, the number of 2D X-ray projections is at least two, in order to provide strong parameters of the error estimations in all directions.

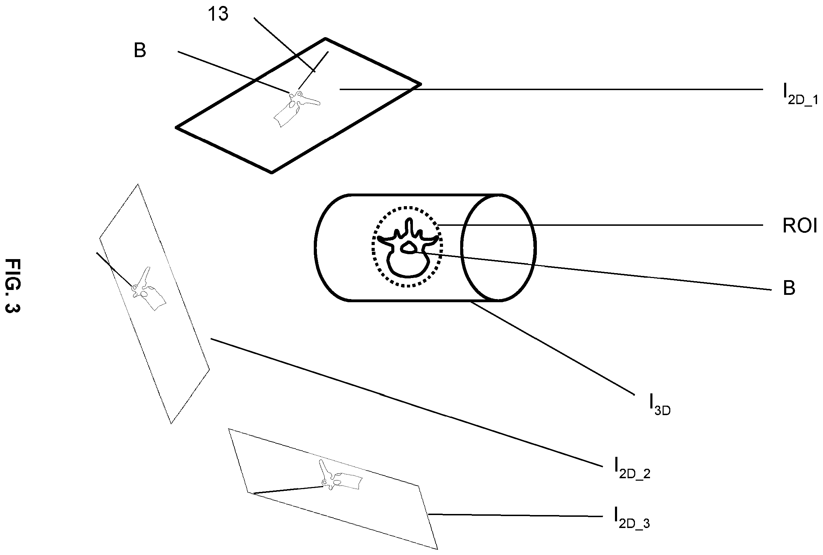

For example, at shown in FIG. 3, three 2D X-ray images I_{2D_1}, I_{2D_2}, I_{2D_3} are acquired with different orientations of the X-ray imaging system. Each 2D X-ray image contains the anatomical structure B and the surgical tool 13.

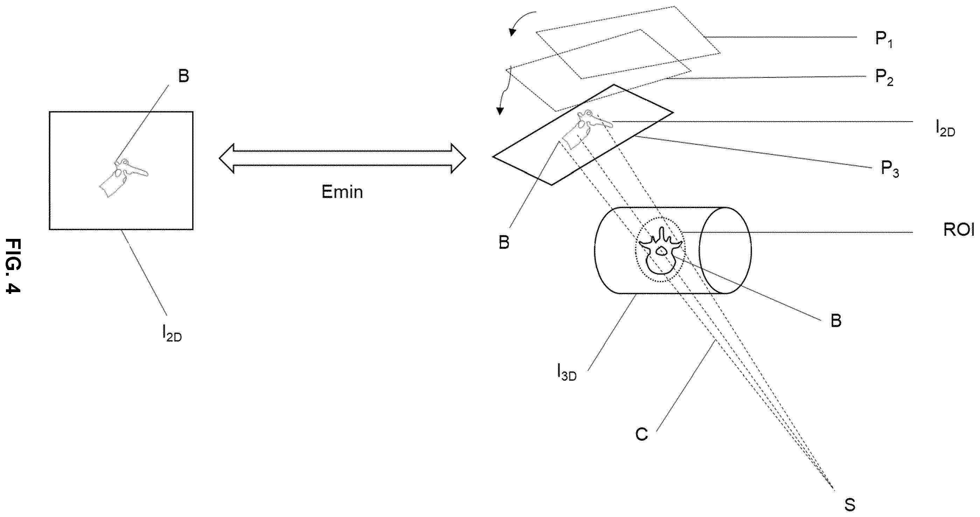

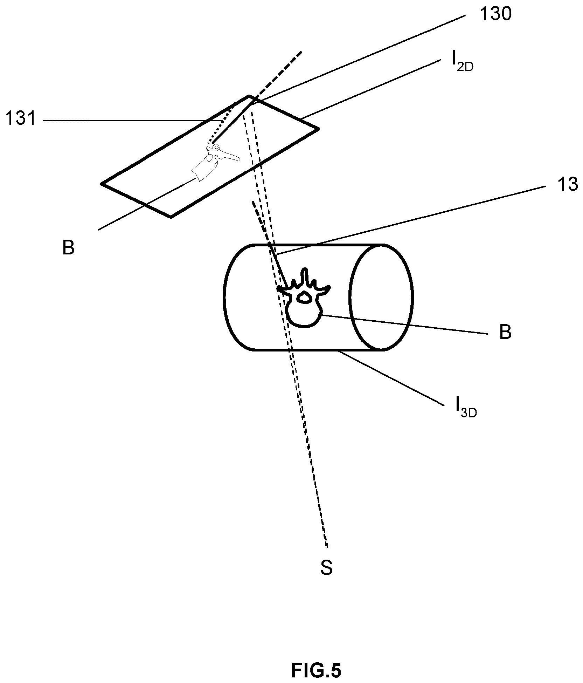

Then the following method is applied:
(a) For each of the at least one 2D X-ray image acquisition, the following method is applied:
   (i) The surgical tool position and the patient tracker are acquired simultaneously with the 2D X-ray image acquisition in order to determine the position of the surgical tool relative to the 3D image.
   (ii) The 2D X-ray image is registered with the 3D image in a region of interest around the anatomical structure using a 3D/2D image registration technique (see FIG. 4 described below),
   (iii) A model of the surgical tool in its position relative to the 3D image is computed (called "projected localized position"),
   (iv) The real position of the surgical tool is determined on the 2D X-ray image (called "real position") (see FIG. 5 described below).
(b) From each 2D X-ray image, a safety criterion is determined from a similarity information between each real position and each projected localized position of the surgical tool on each 2D X-ray images. If several 2D X-ray images are used, a global safety criterion which is a function of all individual similarity information calculated on each 2D X-ray image may be computed.

In a preferred embodiment, said global similarity criterion is the maximum of the similarity information detected on each X-ray image, wherein the similarity information is a distance between the real position and the projected localized position.

If the safety criterion is below a predefined threshold, the robotic system can start autonomously manipulating the surgical tool inside the bone along its planned trajectory.

The method is then repeated all along the path of the robot trajectory, at various instants.

As shown in FIG. 4, the technique for registration of 2D X-ray image I_{2D} with the 3D image I_{3D} can use many algorithms based on the estimation of the position and orientation of the cone C that represents the 2D X-ray image projection with respect to the 3D image by maximizing a similarity criterion between a projection of the 3D image in said position and orientation onto an image plane P₁, P₂, P₃, and the real 2D X-ray image I_{2D}, such algorithms being for example described in [2]. If the anatomical structure belongs to a set of multiple bones, the method preferably selects a region of interest ROI around the anatomical structure in the 3D image I_{3D} and the registration process is applied only to such region of interest. Such a region of interest can be automatically derived from the positions of the surgical tools planned onto the 3D image.

In a preferred embodiment, the X-ray imaging system is a CBCT X-ray imaging system that has been used to acquire the 3D image at the beginning of surgery. The 3D image is acquired in the coordinate system of the patient tracker. The base of the CBCT X-ray imaging system remains fixed on the floor and therefore the patient tracker position can be estimated in the X-ray imaging system.

In this preferred embodiment, the registration process between the 3D image and the X-ray imaging system is initiated by an estimation of the position of the X-ray imaging system. This has the advantage of making the registration process faster and more reliable.

Once the registration between a 2D X-ray image and the 3D image has been applied, it can be reasonably assumed that the position of the anatomical structure does not vary significantly from its previous position, neither the base of the X-ray imaging system.

Therefore, it is possible to use the previously calculated transform between the base of the X-ray imaging system and the 3D image, and the displacement of the X-ray imaging system using the motor encoders values to determine an initial transform, which is used to estimate the precise updated registration between the current X-ray imaging system position and the 3D image. This makes the registration step reliable and fast.

In another embodiment, a third tracker (not shown) can be rigidly attached to and calibrated with the X-ray imaging system. In such case, the initial position of the X-ray imaging system with respect to the 3D image is determined by a simple measurement of the relative patient and X-ray imaging system trackers positions, which makes the registration process faster and more reliable.

FIG. 5 schematically illustrates steps (iii) and (iv) of the method. The projected position in the 2D image I_{2D} of the surgical tool 13 localized in the 3D image I_{3D} is designated by reference 130 (S designating the X-ray source). The real position of the surgical tool in the 2D X-ray image is designated by reference 131.

The comparison of the real position and the projection localized position of the surgical tool in the 2D X-ray image allows determining a safety criterion. Said comparison provides the above-mentioned similarity information.

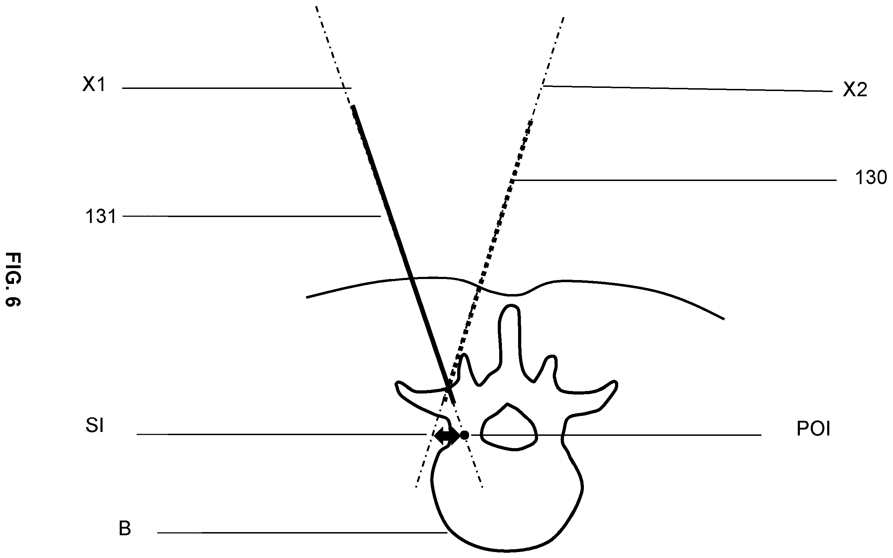

In a preferred embodiment illustrated in FIG. 6, the similarity information SI is the distance between the longitudinal axis X1 corresponding to the real position 131 of the surgical tool 13 and the longitudinal axis X2 corresponding to the projected localized position of the surgical tool on a plane perpendicular to a predetermined point of interest POI along the planned trajectory of the surgical tool.

In a preferred embodiment, the robotic arm advances the surgical tool slowly inside the bone, for example at a speed of one or two millimeters per second. Once a small distance has been performed, such as two millimeters for example, the robotic arm is stopped on its position, and still servo-controlled to track the bone motion in real time, and then the method described above is repeated. A number of 2D X-ray images are acquired and a similarity information is computed to reflect the error between the real surgical tool position and the planned surgical tool position on each projection, and if the maximum error exceeds a predetermined threshold, the robotic arm is stopped and the user is informed that something is wrong and must be modified.

On the contrary, if the maximum error is below a predetermined threshold, the robotic arm may continue to manipulate the surgical tool along the trajectory until it reaches the end of the trajectory.

In another preferred embodiment, the robotic arm is moving continuously along its trajectory without stopping. Several 2D X-ray images are acquired along this motion and for each 2D X-ray image, the method described above is applied. The X-ray imaging system can alternate between two successive positions such that each pair of positions gives an information representative of potential errors in three dimensions even though only 2D projections are acquired. In such embodiment, the X-ray imaging system alternates at a fast rate between two successive positions, for example in less than one second between two positions, and the surgical tool is moved by the robot in the bone at a low speed such as one millimeter per second for example, such that it is possible to check the robot position on the 3D image using two projections every two seconds, which means every two millimeters. If the robot speed is restricted and cannot exceed one millimeter per second, an error on the surgical tool position will be automatically detected such that it cannot exceed two millimeters, which might be an accepted safety limit in some cases.

In another preferred embodiment, once the method steps (a) to (b) have been applied when the surgical tool is placed at the entry point on the bone, the robotic arm advances the surgical tool along its trajectory and the X-ray imaging system stays stable in the same position as previously. Then, only one 2D X-ray image is acquired and the steps (a) to (b) of the method are applied using one 2D X-ray image. In such embodiment, the registration between the 3D image and the X-ray projection benefits from an initial position that is the previous position, making the registration method faster and more reliable despite the fact that there is only one projection.

In another preferred embodiment, once the method steps (a) to (b) have been applied when the surgical tool is placed at the entry point on the bone, the robotic system is moved along its trajectory and the X-ray imaging system stays stable in the same position as previously. A new 2D X-ray image containing the anatomical structure and the surgical tool is acquired and compared to the previously acquired 2D X-ray image. Such comparison is performed by first applying a 2D/2D image registration technique to compensate for any translations or rotations inside the image plane and then calculating a similarity criterion between the two 2D images that have been realigned. Such a similarity criterion can be a correlation coefficient between the 2D X-ray images, a calculation of their mutual entropy or any other image difference criterion known in the art.

Such calculation is performed such that the motion of the surgical tool in the image due to its normal progression shall not impact the measurements. For example, the area in the image around the expected tip of the surgical tool can be removed from the image comparison. If the similarity criterion is below a predefined threshold, a new 2D image is acquired for the same position of the X-ray imaging device and the method is repeated. If the similarity criterion is above said predefined threshold, it can be that there is a real safety issue in the robotic system but it can also happen that the bone has moved in parameters outside of the three image plane parameters but that the robot has successfully tracked the bone position. To distinguish between the two cases, it is necessary to apply the method from the beginning.

In a preferred embodiment, the robotic arm is stopped and servo-controlled on its last position, then at least two 2D X-ray images are acquired and used for comparison with the expected projections once the 2D X-ray images have been registered with the 3D image. If the error is above a given threshold, the robotic arm is stopped and a message is sent to the user that there is an error in the robotic system and that an action must be taken to continue and resume or abandon the process. If the error is below said given threshold, the robotic arm can continue manipulating the tool and a single new 2D X-ray image is acquired and compared to the previously acquired 2D image, and the method is repeated until the robot reaches a final point on the planned trajectory.

In the method described above, the position of the X-ray imaging system with respect to the anatomical structure can be any position. Intuitively, the surgeon might position the X-ray imaging system such that the corresponding 2D X-ray image is clinically relevant and meaningful. For example, a perfect profile view of a spine or a view along the pedicle axis of a vertebra may be clinically meaningful. However, the robotic system can also be used to compute an optimal view and the corresponding orientation of the 2D X-ray imaging system. An optimal 2D X-ray image orientation can be determined to match one or several of the following criteria, partially or totally:
- An orthogonal X-ray projection to the surgical tool axis is optimal in order to get a clear and complete image of the tool, contrary to an image along the axis of the surgical tool in which the projection of the surgical tool would be reduced to a point;
- Considering an intervention on a vertebra, a 2D X-ray image projection in a pure cranio-caudal direction is not possible but a projection in a direction that is at forty five degrees with respect to the longitudinal axis of the patient would allow to detect an error of the robot that would place the surgical tool outside of the pedicle of the vertebra, either in the spinal canal, or outside the pedicles and close to vessels or nerves. Therefore, such an orientation of the X-ray imaging system would be optimal to detect errors with high sensitivity.

Depending on the application, it is therefore possible to determine at least one optimal orientation of the X-ray imaging system relative to either the surgical tool or the anatomical structure and to acquire the at least one 2D X-ray image with said at least one optimal orientation of the X-ray imaging system. Once such optimal position has been determined, the system can move automatically the motorized X-ray imaging system to reach such optimal position.

With the proposed method, the number of 2D X-ray images acquired can be significant to check the position of the robot at a sufficient rate to avoid any possible deviations from the planned trajectory greater than a maximum distance. In order to reduce the X-ray dose that will be applied to the surgical staff, it is possible to use a remote control of the robot and the staff can be protected behind transparent or plain walls that stop X-rays. For example, a joystick with buttons can be used to control the robotic system in all steps of the procedure.

However, the patient may receive many X-ray shots and might be exposed to excessive radiation. To tackle this problem, it is proposed, according to a preferred embodiment of the disclosure, to collimate the X-ray beam on the region of interest around the anatomical structure. Using the nominal parameters of a motorized X-ray imaging system, it is possible to predict the location of the X-ray image with respect to the anatomical structure. Therefore, it is possible to set the collimation of the device such that only the predicted area of the anatomical structure and the planned position of the surgical tool may be visible on the image.

### Application of the method to drilling a linear trajectory in a spine pedicle.

In this embodiment, the surgical target is a linear drilling path in an anatomical structure such as a line in the pedicle of a vertebra for example, or any other drilling line in a bone. The robotic arm holds a surgical drill. In a preliminary approach that does not necessarily require an external safety check, the tip of the drill bit is brought in contact with an entry point of the drilling path on the bone.

Using the external localization system, the robotic system is servo-controlled on the patient tracker and compensates in real-time the motion of the bone, which can be due to patient's breathing, any motion created by the drill itself, or any other interaction on the bone.

For this initial position of the robotic arm, with the drill bit in contact with the entry point, N 2D X-ray images may be acquired for various orientations of the imaging device, wherein N is an integer strictly greater than 1. The bone may move between the N different X-ray image acquisitions. At the instant of each image acquisition, the surgical tool held by the robotic arm and the patient tracker are localized synchronously with the image acquisition. If the robot tracker is directly mounted on the surgical tool, this consists simply in measuring the tracker position attached to the surgical tool. If the robot tracker is attached to a part of the robotic system for which a kinematic model is known with respect to robot encoders values, for example, the base of the robot, it is necessary to apply the transform between the base of the robotic system and the surgical tool using the encoders values of the robotic arm and its kinematic model and to combine it with the measurement of the tracker mounted on the base of the robotic system.

N relative measurements between the surgical tool and the patient tracker are obtained, simultaneously with each of the respective N 2D X-ray image acquisitions. As the transform between the patient tracker and the 3D image is known, by applying this transform, the position of the surgical tool with respect to the 3D image is supposedly known.

But this position may contain errors for many reasons, such as unexpected motion of the patient tracker relative to the anatomical structure, or an error in the measurements of the localization system, due to faulty trackers or a miscalculation in the kinematic model of the robotic arm.

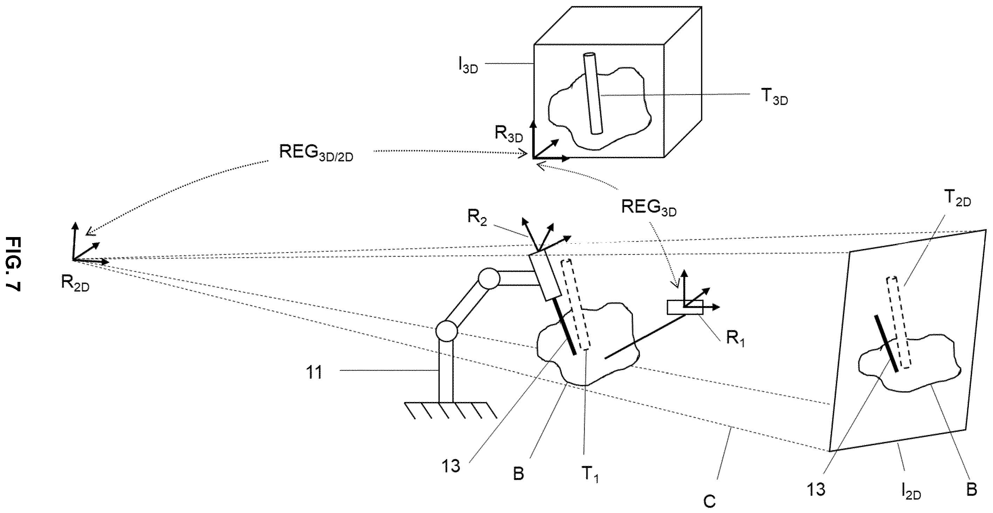

To determine a safety criterion, the following method is applied, with reference to FIG. 7.

Several coordinate systems are involved in this method: R_{3D} is a coordinate system attached to the 3D image, R_{2D} is a coordinate system attached to the 2D image R1 is a coordinate system attached to the patient tracker, R2 is a coordinate system attached to the surgical tool.

The 3D image is registered with the patient tracker R1 by a method described previously: the corresponding transform is designated by REG_{3D}.

Each 2D X-ray image I_{2D} is registered with the 3D image I_{3D}. This is performed using known algorithms of 3D/2D registration between a 3D image and a projection of such 3D image on a 2D plane. The X-ray image projection model is usually known by prior calibration of the imaging device, said calibration provides a geometric model of the X-ray source and the image plane in a coordinate system attached to the X-ray imaging system and usually a cone geometry of the X-ray imaging system is used as a realistic model. Such calibration can take into account the position and orientation of the imaging device since there is a deformation of the relative position of the imaging panel with respect to the X-ray source that depends on the orientation of C-arm. The 3D/2D registration algorithm computes the transform (designated by REG_{3D/2D}) between the coordinate system R_{3D} attached to the 3D image and the coordinate system R_{2D} attached to the X-ray imaging system.

Then, a projection T_{2D} of the planned linear trajectory T_{3D} known in the 3D image can be generated onto the 2D X-ray image I_{2D} using the resulting transform.

The planned trajectory of the surgical tool in in the coordinate system R1 is designated by T1.

Furthermore, the real position of the surgical tool 13 in the 2D X-ray image I_{2D} is determined, by identifying for example the straight line corresponding to the drill axis.

Finally, a safety criterion is determined from a similarity information between the real position of the surgical tool in the 2D X-ray image and the projected localized position of the surgical tool in said 2D X-ray image.

In a preferred embodiment, the similarity information is the distance between the two lines calculated at the level of a point of interest defined on the planned trajectory. For example, it can be a point of the linear trajectory that is in the center of the pedicle of a vertebra because this point is the most sensitive for the safety of the operation.

It can be noted that using only one projection in such 3D/2D registration gives an accurate estimation of three parameters of this transform (strong parameters) and a less accurate estimation of the three other parameters of this transform (weak parameters), but this well-known phenomenon does not affect significantly the accuracy of the proposed method because the influence of the weak parameters on the calculation of the projection of a point or line does not shift significantly the projected point or line itself.

Then, the robotic arm is moved by its controller to advance the surgical drill onto the linear trajectory, inside the bone, starting actual drilling, with an advancing speed of two millimeters per second.

The X-ray imaging system remains in a fixed position.

At any instant during the progression of the surgical drill along its linear path, an X-ray image may be acquired, and the positions of the patient tracker and robot tracker may be measured with a high degree of synchronization, within a few milliseconds. The method described above is applied to compute a safety criterion that indicates if the surgical drill is correctly following the linear trajectory within a threshold of one millimeter for example. The images are iteratively acquired every 0.5 seconds and the safety criterion is calculated for each X-ray image. For reducing the generated X-ray dose, the images are collimated to a small area of less than ten by ten centimeters which is sufficient to visualize and measure the vertebra and the surgical drill axis.

The process is repeated until the drill reaches a planned end point on the linear trajectory.

### Application of the method to burring a volume in a bone.

In this embodiment, the surgical target is a bone volume that requires to be resected using a surgical burr. For instance said volume may be the volume to be resected to decompress the spinal canal or remove a bone tumor, or said volume may be the volume to be resected on a femur for preventing a femoro-acetabular impingement of the hip.

Typically, for applications demanding a very high accuracy, the volume to be resected is in the range of a few cubic millimeters and the burr is spherical with a diameter between 1mm and 5mm.

Once a resection volume has been defined, a burring 3D complex path is planned in the 3D image. Said path contains a starting point and an end point, and a list of successive points between said starting point and end point. Said path is optimized using conventional robotics path planning techniques, depending on multiple parameters such as the size of the burr, the desired robotic system speed and the burr sharpness.

The robotic arm holds a surgical burr. In a preliminary approach that does not necessarily require an external safety check, the tip of the burr is brought in contact with a starting point of the burring path on the bone.

Using the external localization system, the robotic arm is servo-controlled on the patient tracker and compensates in real-time the motion of the bone, which can be due to patient's breathing, any motion created by the drill itself, or any other interaction on the bone.

For this initial position of the robotic arm, the burr being in contact with the starting point, two 2D X-ray images are acquired for some selected orientations of the imaging device. Synchronously, for each 2D X-ray image, the position of the burr relative to the 3D image is measured with the localization system. And the method described above is applied to check that the surgical burr is precisely at its expected position on the bone. If not, the robot is stopped.

Once the first safety check has passed, the powered burr is activated at high speed and the robotic system starts executing a burring 3D complex path with a slow advancing speed of 0.5mm per second.

The X-ray imaging system remains in a fixed position. The robotic system executes the planned path thus moving the burr tip from one target point to the next target point along the planned path. For each target point defined in the 3D image, the robotic system is servo-controlled so that the burr reaches the corresponding target point in the coordinate system of the patient tracker, after the 3D registration transform initially calculated between the patient tracker and the 3D image has been applied. At any given instant during the performance of the burring 3D complex path, the surgical burr is therefore supposed to be at a precisely known position in the 3D image.

At any instant, and repeatedly at a frame of ten times per second, a collimated 2D X-ray image is acquired and the patient tracker and robot trackers are localized synchronously, with a time lag between X-ray image acquisition and tracker localization that must not exceed ten milliseconds as a reasonable value. The 2D X-ray image is registered with the 3D image in a given region of interest using the methods described above, and benefiting from all the different techniques that make it reliable and fast, such that the computation of 3D/2D registration does not exceed also a few milliseconds.

The projection of the planned position of the burring tool estimated at this instant on the 3D image is calculated on the image plane and the real image of the burring tool is detected in the real image by searching the precise geometry of the burr tip and axis, which also requires an extremely fast process. The fast process is made possible by the fact that the surgical burr position estimation is known on the 2D image and therefore only small deviations from the expected position are searched for, which is much faster and reliable than searching a burr tool in a full image at any location.

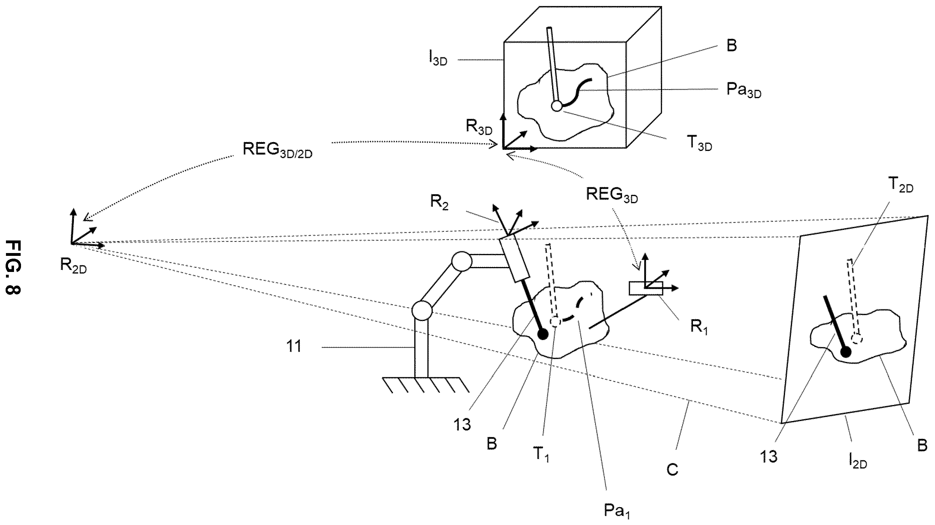

FIG. 8 schematically illustrates the method implemented in this embodiment. The elements identical to the ones of FIG. 7 will not be described again. The difference between these figures is that in FIG. 8 both a planned trajectory of the surgical tool T_{3D}, T1 and a planned 3D complex path Pa_{3D}, Pa1 are determined respectively in the coordinate systems R_{3D} and R₁.

Then a distance is calculated between the projected model of the burr and the real image of the burr. If the burr has a spherical shape, the distances between the centers of the real and projected burrs is calculated. Else, any geometric characteristic of the real burr can be compared to the model of the burr. A subpixel calculation can be performed on the X-ray image, which corresponds to a fraction of a millimeter, for example a tenth of a millimeter can be detected. If the maximum cumulated lag is in the range of twenty milliseconds and the maximum speed of the robotic arm is set by a safe controller to a value of 0.5mm per second, the maximum error induced by the lag measurements is 0.01 mm which is an order of magnitude inferior to the required accuracy measurements for such application. Since the frame rate of image acquisition is ten images per second, the maximum displacement between two frames is 0.05mm to which the error lag of 0.01mm can be added to produce a maximal error of 0.06mm, which is therefore the maximum error of the method. In most application, it is necessary to detect an error of the robot of 1mm, therefore the method accuracy is sufficient.

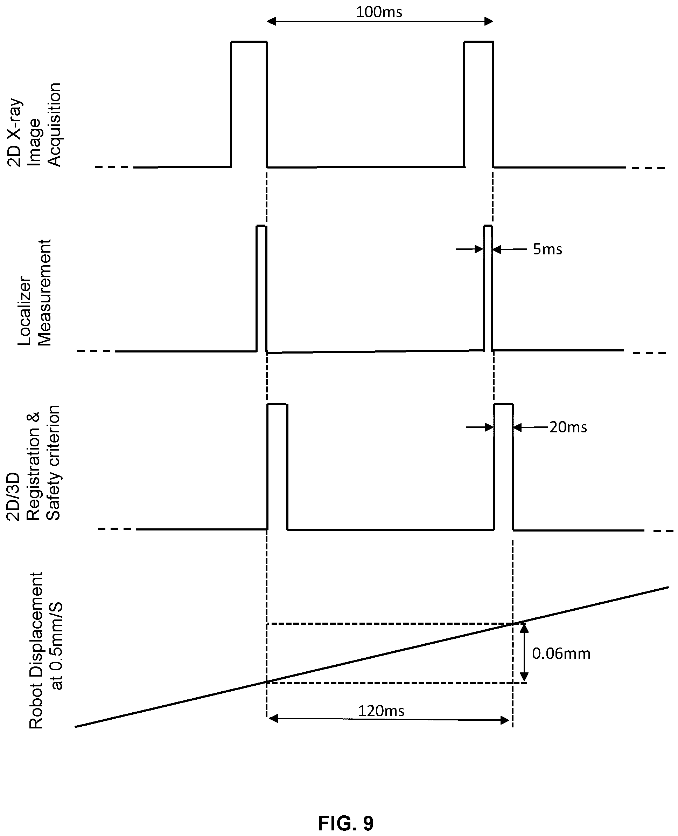

An example of the management of the lags is illustrated schematically in FIG. 9.

All the graphs of FIG. 9 have the same time scale.

From the top to the bottom of FIG. 9, the first graph represents the time slots for acquisitions of 2D X-ray images. For example, two successive acquisitions are separated by an interval of 100ms.

The second graph represents the time slots for measurement of the positions of the trackers by the localization system. These time slots are typically shorter (5ms) than the time slots for the acquisition of a 2D X-ray image (about 15ms). As represented by the dotted line between the first and second graphs, the localization measurement is synchronized with the acquisition of a 2D X-ray image.

The third graph represents the time slots for carrying out the 2D/3D registration and computation of the safety criterion. In the example, these time slots are of about 20ms.

The fourth graph represents the displacement of the robotic arm at a speed of 0.5mm/s. Taking into account the lag due to the computation and the interval between two acquisitions of 2D X-ray images and localization measurements, an error will be detected in at most 120ms, which represents a displacement of 0.06mm of the robotic arm at a speed of 0.05mm.

The skilled person will be able to adjust previous parameters for each application to obtain a reasonable global time of execution of the surgical procedure, in particular robotic system displacement speed, burr diameter and frame of 2D X-ray images and maximal acceptable deviation.

### REFERENCES

[1] Marco Caversaccio, et al., Robotic cochlear implantation: surgical procedure and first clinical experience, Acta Oto-Laryngologica (2017) 137:4, 447-454
[2] P. Markelja et al., A review of 3D/2D registration methods for image-guided interventions, Medical Image Analysis, Volume 16, Issue 3, April 2012, Pages 642-661

## Claims

1. Control unit for determining a safety criterion during an autonomous manipulation of a surgical tool (13) by a robotic system (1) to treat an anatomical structure (B) according to a planned trajectory (T_{3D}) in a 3D image (I_{3D}), said 3D image being registered with a patient tracker (30), and the robotic system (1) being servo-controlled on the movements of the patient tracker (30), the control unit being configured to implement the following method:
a. receive at least one 2D X-ray image (I_{2D}) containing the anatomical structure and the surgical tool by an X-ray imaging system (2), and for each at least one 2D X-ray acquisition:
i. synchronously localize the surgical tool and the patient tracker to determine the position of the surgical tool relative to said 3D image,
ii. registering the 2D X-ray image (I_{2D}) with the 3D image (I_{3D}) in a region of interest around the anatomical structure,
iii. generating a projection onto the 2D X-ray image (I_{2D}) of a model of the surgical tool in its position relative to the 3D image computed in step (i) ('projected localized position'),
iv. determining a real position of the surgical tool on the 2D X-ray image (I_{2D}) ('real position'),
b. determining a safety criterion from a similarity information between each real position and each projected localized position of the surgical tool on the at least one 2D X-ray image.

2. Control unit according to claim 1, further configured to repeat steps (a) and (b) several times all along the autonomous manipulation of the surgical tool by the robotic system.

3. Control unit according to claim 1 or claim 2, further configured to compute in step (a) at least one optimal orientation of the X-ray imaging system (2) relative to either the surgical tool or the anatomical structure and to control the X-ray imaging system to acquire the at least one 2D X-ray image with said at least one optimal orientation.

4. Control unit according to any one of claims 1 to 3, further configured to apply each one of steps (a) through (b) to at least two 2D X-ray images (I_{2D_1}, I_{2D_2}) and to compute in step (b) a global safety criterion as a function of each similarity information computed for each 2D X-ray image.

5. Control unit according to claim 4, wherein said function is the maximum function.

6. Control unit according to any one of claims 1 to 5, further configured to initiate the registration process in step (ii) by a known estimation of the position of the X-ray imaging system (2).

7. Control unit according to any one of claims 1 to 6, wherein the similarity information is the distance between the longitudinal axis corresponding to the real position of the surgical tool and the longitudinal axis corresponding to its projected localized position on a plane perpendicular to a predetermined point of interest along the planned trajectory of the surgical tool.

8. System for an autonomous manipulation of a surgical tool to treat an anatomical structure according to a planned trajectory in a 3D image, said 3D image being registered with a patient tracker, said system comprising:
- a robotic system (1) configured to be servo-controlled on the movements of the patient tracker (30), the robotic system (1) being configured to manipulate the surgical tool (13) according to the planned trajectory,
- an X-ray imaging system (2) configured to acquire at least one 2D X-ray image containing the anatomical structure (B) and the surgical tool (13),
- a localization system (3) adapted to measure relative positions of the patient tracker and of a tracker rigidly fixed to the surgical tool or to a part of the robotic system,
- the control unit according to any one of claims 1 to 7 coupled to the robotic system, the X-ray imaging system and the localization system.

9. System according to claim 8, further comprising a tracker rigidly fixed to a part of the robotic system and adapted to be tracked by the localization system (3), the control unit being configured to determine a localization of the surgical tool from localization measurement of the tracker rigidly fixed to the part of the robotic system and from a known kinematic model of the robotic system between said tracker and the surgical tool at the time of localization.

## Patentansprüche

1. Steuereinheit zum Bestimmen eines Sicherheitskriteriums während einer autonomen Manipulation eines chirurgischen Werkzeugs (13) durch ein Robotersystem (1), um eine anatomische Struktur (B) gemäß einer geplanten Trajektorie (T_{3D}) in einem 3D-Bild (I_{3D}) zu behandeln, wobei das 3D-Bild bei einem Patientenverfolger (30) registriert ist und wobei das Robotersystem (1) anhand der Bewegungen des Patientenverfolgers (30) servogesteuert ist, wobei die Steuereinheit dazu ausgelegt ist, das folgende Verfahren zu implementieren:
a. Empfangen von mindestens einem 2D-Röntgenbild (I_{2D}), das die anatomische Struktur und das chirurgische Werkzeug enthält, durch ein Röntgenbildgebungssystem (2) und für jede mindestens eine 2D-Röntgenerfassung:
i. synchrones Lokalisieren des chirurgischen Werkzeugs und des Patientenverfolgers, um die Position des chirurgischen Werkzeugs relativ zum 3D-Bild zu bestimmen,
ii. Registrieren des 2D-Röntgenbildes (I_{2D}) beim 3D-Bild (I_{3D}) in einer Region von Interesse um die anatomische Struktur,
iii. Erzeugen einer Projektion eines Modells des chirurgischen Werkzeugs in seiner Position relativ zum 3D-Bild, die in Schritt (i) (,projizierte lokalisierte Position') berechnet wurde, auf das 2D-Röntgenbild (I_{2D}),
iv. Bestimmen einer realen Position des chirurgischen Werkzeugs auf dem 2D-Röntgenbild (I_{2D}) (,reale Position'),
b. Bestimmen eines Sicherheitskriteriums anhand von Ähnlichkeitsinformationen zwischen jeder realen Position und jeder projizierten lokalisierten Position des chirurgischen Werkzeugs auf dem mindestens einen 2D-Röntgenbild.

2. Steuereinheit nach Anspruch 1, die ferner dazu ausgelegt ist, die Schritte (a) und (b) zusammen mit der autonomen Manipulation des chirurgischen Werkzeugs durch das Robotersystem mehrere Male zu wiederholen.

3. Steuereinheit nach Anspruch 1 oder Anspruch 2, die ferner dazu ausgelegt ist, in Schritt (a) mindestens eine optimale Ausrichtung des Röntgenbildgebungssystems (2) relativ entweder zum chirurgischen Werkzeug oder zur anatomischen Struktur zu berechnen und das Röntgenbildgebungssystem zum Erfassen des mindestens einen 2D-Röntgenbildes mit der mindestens einen optimalen Ausrichtung zu steuern.

4. Steuereinheit nach einem der Ansprüche 1 bis 3, die ferner dazu ausgelegt ist, jeden der Schritte (a) bis (b) auf mindestens zwei 2D-Röntgenbilder (I_{2D_1}, I_{2D-2}) anzuwenden und in Schritt (b) ein globales Sicherheitskriterium als eine Funktion von allen Ähnlichkeitsinformationen zu berechnen, die für jedes 2D-Röntgenbild berechnet wurden.

5. Steuereinheit nach Anspruch 4, wobei die Funktion die maximale Funktion ist.

6. Steuereinheit nach einem der Ansprüche 1 bis 5, die ferner dazu ausgelegt ist, den Registrierungsprozess in Schritt (ii) durch eine bekannte Schätzung der Position des Röntgenbildgebungssystems (2) zu initiieren.

7. Steuereinheit nach einem der Ansprüche 1 bis 6, wobei sich die Ähnlichkeitsinformationen auf den Abstand zwischen der Längsachse, die der realen Position des chirurgischen Werkzeugs entspricht, und der Längsachse, die seiner projizierten lokalisierten Position entspricht, auf einer Ebene senkrecht zu einem vorbestimmten Interessenpunkt entlang der geplanten Trajektorie des chirurgischen Werkzeugs beziehen.

8. System für eine autonome Manipulation eines chirurgischen Werkzeugs, um eine anatomische Struktur gemäß einer geplanten Trajektorie in einem 3D-Bild zu behandeln, wobei das 3D-Bild bei einem Patientenverfolger registriert ist, wobei das System Folgendes umfasst:
- ein Robotersystem (1), das dazu ausgelegt ist, anhand der Bewegungen des Patientenverfolgers (30) servogesteuert zu werden, wobei das Robotersystem (1) dazu ausgelegt ist, das chirurgische Werkzeug (13) gemäß der geplanten Trajektorie zu manipulieren,
- Röntgenbildgebungssystem (2), das dazu ausgelegt ist, mindestens ein 2D-Röntgenbild zu erfassen, das die anatomische Struktur (B) und das chirurgische Werkzeug (13) enthält,
- ein Lokalisierungssystem (3), das angepasst ist, relative Positionen des Patientenverfolgers und eines Verfolgers zu messen, der starr am chirurgischen Werkzeug oder an einem Teil des Robotersystems befestigt ist,
- die Steuereinheit nach einem der Ansprüche 1 bis 7, die an das Robotersystem, das Röntgenbildgebungssystem und das Lokalisierungssystem gekoppelt ist.

9. System nach Anspruch 8, das ferner einen Verfolger umfasst, der starr an einem Teil des Robotersystems befestigt und angepasst ist, vom Lokalisierungssystem (3) verfolgt zu werden, wobei die Steuereinheit dazu ausgelegt ist, eine Lokalisierung des chirurgischen Werkzeugs anhand einer Lokalisierungsmessung des Verfolgers, der starr am Teil des Robotersystems befestigt ist, und anhand eines bekannten kinematischen Modells des Robotersystems zwischen dem Verfolger und dem chirurgischen Werkzeug zum Zeitpunkt der Lokalisierung zu bestimmen.

## Revendications

1. Unité de commande pour déterminer un critère de sécurité pendant une manipulation autonome d'un outil chirurgical (13) par un système robotique (1) afin de traiter une structure anatomique (B) selon une trajectoire planifiée (T_{3D} ) dans une image 3D (I_{3D}), ladite image 3D étant recalée avec un dispositif de suivi du patient (30), et le système robotique (1) étant asservi aux mouvements du dispositif de suivi du patient (30), l'unité de commande étant configurée pour mettre en œuvre le procédé suivant :
a. recevoir au moins une image radiographique 2D (I_{2D}) contenant la structure anatomique et l'instrument chirurgical par un système d'imagerie radiographique (2), et pour chaque acquisition radiographique 2D :
i. localiser de manière synchrone l'outil chirurgical et le dispositif de suivi du patient afin de déterminer la position de l'outil chirurgical par rapport à ladite image 3D,
ii. recaler l'image radiographique 2D (I_{2D}) avec l'image 3D (I_{3D}) dans une région d'intérêt autour de la structure anatomique,
iii. générer une projection sur l'image radiographique 2D (I_{2D}) d'un modèle de l'instrument chirurgical dans sa position par rapport à l'image 3D calculée à l'étape (i) (« position localisée projetée »),
iv. déterminer une position réelle de l'instrument chirurgical sur l'image radiographique 2D (I_{2D}) (« position réelle »),
b. déterminer un critère de sécurité à partir d'une information de similarité entre chaque position réelle et chaque position localisée projetée de l'outil chirurgical sur au moins une image radiographique 2D.

2. Unité de commande selon la revendication 1, configurée en outre pour répéter les étapes (a) et (b) plusieurs fois tout au long de la manipulation autonome de l'instrument chirurgical par le système robotique.

3. Unité de commande selon la revendication 1 ou la revendication 2, configurée en outre pour calculer à l'étape (a) au moins une orientation optimale du système d'imagerie radiographique (2) par rapport à l'instrument chirurgical ou à la structure anatomique et pour commander le système d'imagerie radiographique afin d'acquérir au moins une image radiographique 2D avec ladite au moins une orientation optimale.

4. Unité de commande selon l'une quelconque des revendications 1 à 3, configurée en outre pour appliquer chacune des étapes (a) à (b) à au moins deux images radiographiques 2D (I_{2D_1}, I_{2D_2}) et pour calculer à l'étape (b) un critère de sécurité global en fonction de chaque information de similarité calculée pour chaque image radiographique 2D.

5. Unité de commande selon la revendication 4, dans laquelle ladite fonction est la fonction maximale.

6. Unité de commande selon l'une quelconque des revendications 1 à 5, configurée en outre pour lancer le processus de recalage à l'étape (ii) par une estimation connue de la position du système d'imagerie radiographique (2).

7. Unité de commande selon l'une quelconque des revendications 1 à 6, dans laquelle l'information de similarité est la distance entre l'axe longitudinal correspondant à la position réelle de l'outil chirurgical et l'axe longitudinal correspondant à sa position localisée projetée sur un plan perpendiculaire à un point d'intérêt prédéterminé le long de la trajectoire planifiée de l'outil chirurgical.

8. Système pour la manipulation autonome d'un outil chirurgical afin de traiter une structure anatomique selon une trajectoire planifiée dans une image 3D, ladite image 3D étant recalée avec un dispositif de suivi du patient, ledit système comprenant :
- un système robotique (1) configuré pour être asservi aux mouvements du dispositif de suivi du patient (30), le système robotique (1) étant configuré pour manipuler l'outil chirurgical (13) selon la trajectoire planifiée,
- un système d'imagerie radiographique (2) configuré pour acquérir au moins une image radiographique 2D contenant la structure anatomique (B) et l'instrument chirurgical (13),
- un système de localisation (3) adapté pour mesurer les positions relatives du dispositif de suivi du patient et d'un dispositif de suivi fixé de manière rigide à l'instrument chirurgical ou à une partie du système robotique,
- une unité de commande selon l'une quelconque des revendications 1 à 7 couplée au système robotique, au système d'imagerie radiographique et au système de localisation.

9. Système selon la revendication 8, comprenant en outre un traceur fixé rigidement à une partie du système robotique et adapté pour être suivi par le système de localisation (3), l'unité de commande étant configurée pour déterminer une localisation de l'outil chirurgical à partir de la mesure de localisation du traceur fixé rigidement à la partie du système robotique et à partir d'un modèle cinématique connu du système robotique entre ledit traceur et l'outil chirurgical au moment de la localisation.
